# EUROPÄISCHE PATENTSCHRIFT

(11) **EP 0 487 852 B1**
(45) Veröffentlichungstag und Bekanntmachung des Hinweises auf die Patenterteilung: **10.08.1994**
(21) Anmeldenummer: 91115992.9
(22) Anmeldetag: 20.09.1991
(51) Int. Cl.: A61F 2/66

(54) **Gelenkloser Prothesenfuss**
Jointless prosthetic foot
Pied prothétique non articulé

(30) Priorität: 29.11.1990 DE 4038063
(43) Veröffentlichungstag der Anmeldung: 03.06.1992
(73) Patentinhaber: Otto Bock Orthopädische Industrie Besitz- und Verwaltungs-Kommanditgesellschaft, 37115 Duderstadt (DE)
(72) Erfinder: Wellershaus, Ulf,Orthopädie-Mechanikermeister, W-3408 Duderstadt (DE)
(74) Vertreter: Gramm, Werner, Prof., Dipl.-Ing.

(56) Entgegenhaltungen:
- EP-A- 0 331 468
- EP-A- 0 401 864
- DE-C- 325 171
- FR-A- 2 640 499
- US-A- 2 453 969
- US-A- 4 645 509
- US-A- 4 652 266

## Beschreibung

Die Erfindung betrifft einen gelenklosen Prothesenfuß mit einem innerhalb des Prothesenkörpers vorgesehenen, die Prothesenbelastungen aufnehmenden und übertragenden federelastischen, einteilig ausgebildeten Fußeinsatz, der einen oberen horizontalen, den oberen Abschluß des Prothesenfußes bildenden und eine Verbindungsmöglichkeit mit der Prothese bietenden Schenkel sowie einen unteren Schenkel aufweist, der gegenüber dem oberen Schenkel verlängert ausgebildet ist und sich mit seinem freien Ende bis in den Fußspitzenbereich hinein erstreckt zur Erzielung einer hohen Vorfußelastizität in Verbindung mit einem hohen Energiespeichervermögen bei Vorfußbelastung.

Eine entsprechende Ausführungsform ist durch den sogenannten Seattle-Fuß bekanntgeworden (siehe VETERANS ADMINISTRATION - JOURNAL OF REHABILITATION RESEARCH AND DEVELOPMENT, Bd. 22, Nr.3 BPR 10-42, Seiten 75-84, insbesondere Abbildung 6 oder US-A-4,645,509).

Die Funktion eines Prothesenfußes hängt im wesentlichen von seinen elastischen Eigenschaften ab. Diese werden bestimmt durch Material, Gestalt und Anordnung der verwendeten elastischen Komponenten.

Je nach vorgesehenem Einsatzbereich, der sich je nach Neigungen des Amputierten vom normalen Gehen in der Ebene bis zum sportlichen Einsatz wie Joggen, Laufen, Springen erstrecken kann, sind die Anforderungen an solche Komponenten sehr unterschiedlich, insbesondere was ihr Arbeitsvermögen und der eng damit im Zusammenhang stehende Verlauf ihrer Federkennlinien bei Be- und Entlastung betrifft.

Die jüngeren Entwicklungen gelenkloser Prothesenfüße für einen möglichst weiten Einsatzbereich weichen vom klassischen Konzept des SACH-Fußes dadurch ab, daß sie den starren Fußkern im Vorfußbereich mit Federelementen ergänzen oder ihn ganz durch solche ersetzen (z.B. SEATTLE-Fuß), um die Vorfußelastizität und damit auch das Energiespeichervermögen bei Vorfußbelastung zu verbessern. Die Fersenelastizität wird dabei konzeptionell weitgehend unverändert durch einen Schaumstoffkeil im Fersenbereich erreicht.

Der Erfindung liegt die Aufgabe zugrunde, einen Prothesenfuß der eingangs erläuterten Ausführungsart mit verbesserten Eigenschaften zu entwickeln.

Diese Aufgabe wird gemäß der Erfindung dadurch gelöst, daß der zumindest eine Plantar- und Dorsalflexion sowie eine axiale Kompression ermöglichende Fußeinsatz im Fußlängsschnitt eine angenähert S-förmige Ausbildung aufweist, wobei der genannte obere Schenkel mit einem sich unter einem stumpfen Winkel anschließenden vorderen Schrägschenkel ein insgesamt starres Winkelelement bildet, an dessen unteres Ende sich ein mittlerer, blattfederähnlicher Schenkel anschließt, der an seinem hinteren Ende über eine angenähert halbkreisförmige Schenkelverbindung mit dem unteren Schenkel verbunden ist.

Zur Erzielung eines natürlicheren Bewegungsablaufes für den Prothesenträger ist es zweckmäßig, wenn sich das untere Ende des starren Winkelelementes nach vorn bis etwa in den Bereich der Zehengrundgelenke erstreckt.

Um bei zunehmenden Vorfußwinkel die Biegung des unteren Schenkels des Fußeinsatzes weiter nach vorn zu verlagern, ist es vorteilhaft, wenn der untere Schenkel des Fußeinsatzes unterhalb des Anschlußbereiches des mittleren Schenkels am unteren Ende des starren Winkels eine obere Auswölbung aufweist, die mit der oberhalb von ihr liegenden Fläche des genannten Anschlußbereiches einen sich nach vorn leicht keilfömig öffnenden Luftspalt einschließt.

Erfindungsgemäß soll der Fußeinsatz so ausgebildet sein, daß der obere Schenkel des Fußeinsatzes in einer Horizontalebene gegenüber dem unteren Schenkel verwindbar ist, also eine Torsion und dadurch eine Rotation des Prothesenfußes um eine vertikale Achse zuläßt. Dabei ist es ferner vorteilhaft, wenn der obere Schenkel des Fußeinsatzes in der lotrecht und quer zur Gehrichtung liegenden Frontalebene gegenüber dem unteren Schenkel verwindbar ist zur Anpassung an Neigungen quer zur Gehrichtung. Erfindungsgemäß ist es möglich, daß die die Kraft-Weg- bzw. Winkel-Moment-Charakteristik der die genannten Verschwenkungen bzw. Verformungen bestimmenden federelastischen Eigenschaften (Federkennlinien) durch den S-fömigen Fußeinsatz allein bestimmt sind. Jedoch können die genannten Federkennlinien auch im Zusammenwirken des S-fömigen Fußeinsatzes mit zusätzlichen federelastischen Komponenten erzielt werden.

Die Plantarflexion wird durch Verkleinerung des zwischen dem vorderen Schrägschenkel und dem mittleren Schenkel eingeschlossenen Winkels in einer Ebene senkrecht zum Becken und parallel zur Laufrichtung erreicht. Die Dorsalflexion ergibt sich durch Verkleinerung des Abstandes des unteren Endes des vorderen Schrägschenkels von der Oberfläche des unteren Schenkels des Fußeinsatzes.

Um unter Belastung ein zu großes Aufspreizen des oberen Schenkels des Fußeinsatzes gegenüber dem als Blattfeder wirkenden mittleren Schenkels zu verhindern, ist es vorteilhaft, wenn für das freie Ende des oberen Schenkels des Fußeinsatzes ein Biegebegrenzer vorgesehen ist.

Der Prothesenkörper bildet vorzugsweise eine einteilige, alle Funktionselemente umschließende kosmetische Hülle, die äußerlich keinerlei Befestigungselemente aufweist. Dabei kann der Prothesenkörper so ausgebildet sein, daß er im letzten Bereich der Standphase für den Fußeinsatz eine teilweise Stützfunktion bietet. Insbesondere ist es vorteilhaft, wenn der Prothesenkörper mit Ausnahme seiner oberen Anschlußfläche vollständig von einer hautbildenden äußeren Kunststoffschaumschicht ummantelt ist, die einen sich bis in den Zehenbereich erstreckenden Innenfuß aus Kunststoffschaum umschließt.

Weitere Merkmale der Erfindung sind Gegenstand der Unteransprüche und werden in Verbindung mit weiteren Vorteilen der Erfindung anhand von Ausführungsbeispielen näher erläutert.

In der Zeichnung sind einige als Beispiele dienende Ausführungsformen der Erfindung dargestellt. Es zeigen:
- Figur 1: einen vertikalen Längsschnitt durch einen gelenklosen Prothesenfuß mit einem federelasischen Fußeinsatz;
- Figur 2: den Fußeinsatz gemäß Figur 1 in separater Darstellung;
- Figur 3: den Prothesenfuß gemäß Figur 1 in einer Belastungsphase und
- Figur 4: eine abgewandelte Ausführungsform in einer Darstellung gemäß Figur 1.

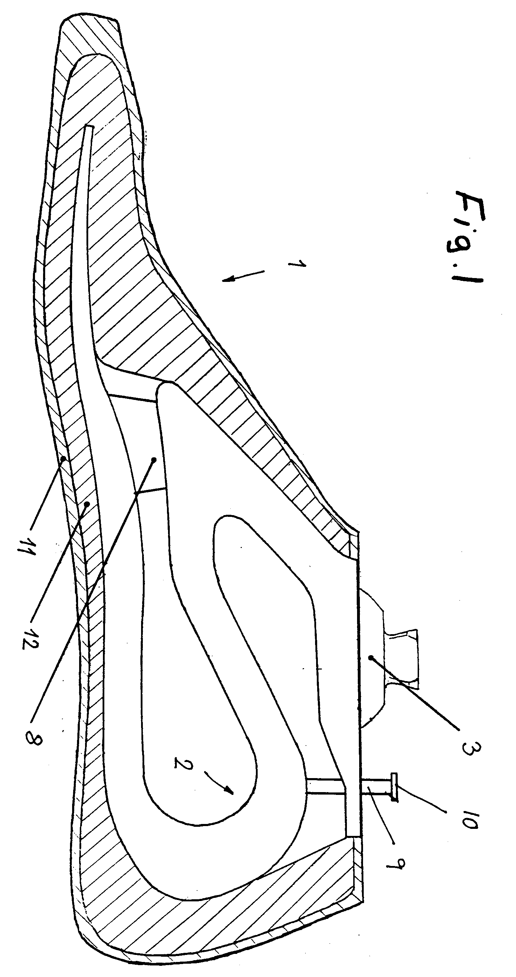

Der in Figur 1 dargestellte gelenklose Prothesenfuß weist einen Prothesenkörper 1 auf, der einen die Prothesenbelastungen aufnehmenden und übertragenden federelastischen Fußeinsatz 2 umschließt, der einteilig ausgebildet ist und zumindest eine Plantar- und Dorsalflexion sowie eine axiale Kompression ermöglicht.

Der Fußeinsatz 2 weist im Fußlängsschnitt gesehen eine angenähert S-fömige Ausbildung auf. Der obere horizontale Schenkel 2a bildet den oberen Abschluß des Prothesenfußes und ist mit einem Fußadapter 3 versehen zur lösbaren Verbindung mit einer Prothese. Dieser obere Schenkel 2a bildet mit einem sich unter einem stumpfen Winkel anschließenden vorderen Schrägschenkel 2b ein insgesamt starres Winkelelement, an dessen unteres Ende 4 sich ein mittlerer, blattfederähnlicher Schenkel 2c anschließt. Dieser ist an seinem hinteren Ende über eine angenähert halbkreisförmige Schenkelverbindung 2d mit einem unteren Schenkel 2e verbunden, der gegenüber dem oberen Schenkel 2a verlängert ausgebildet ist und sich mit seinem freien Ende bis in den Fußspitzenbereich hinein erstreckt zur Erzielung einer hohen Vorfußelastizität in Verbindung mit einem hohen Energiespeichervermögen bei Vorfußbelastung. Die untere Kontur des unteren horizontalen Schenkels 2e ist an die Kontur der Fußunterseite angepaßt. Ferner weist der untere Schenkel 2e unterhalb des Anschlußbereiches des mittleren Schenkels 2c am unteren Ende 4 des starren Winkels 2a, 2b eine obere Auswölbung 5 auf, die mit der oberhalb von ihr liegenden Fläche 6 des genannten Anschlußbereiches einen sich nach vorn leicht keilförmig öffnenden Luftspalt 7 einschließt. Dabei erstreckt sich das untere Ende 4 des starren Winkelelementes 2a, 2b nach vorn bis etwa in den Bereich der Zehengrundgelenke.

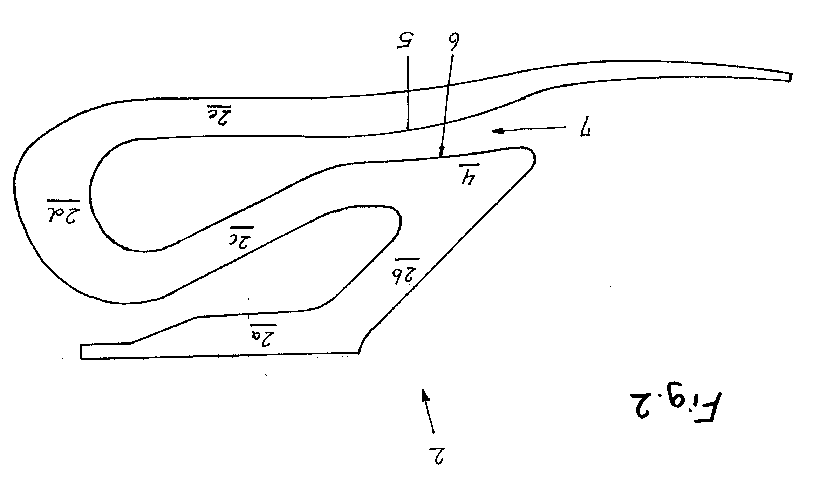

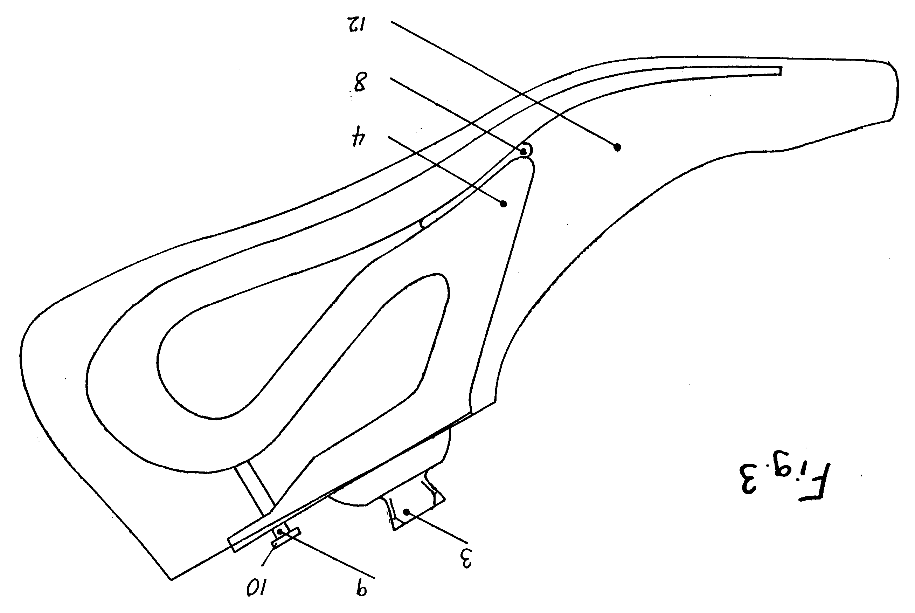

Das starre Winkelelement 2a, 2b stützt sich an seinem unteren Ende 4 über ein federelastisches Element 8 auf dem unteren Schenkel 2e ab. Dieses federelastische Element 8 wird bei Belastung zusammengedrückt, wie Figur 3 erkennen läßt. Bei der in den Figuren 1 bis 3 dargestellten Ausführungsform ist die hintere Schenkelverbindung 2d des Fußeinsatzes 2 starr ausgebildet. Der Fußeinsatz 2 setzt sich also zusammen aus einem weitgehend starr ausgebildeten Winkelteil 2a, 2b, einer mittleren Blattfeder (mittlerer Schenkel 2c), einer unteren Blattfeder (unterer Schenkel 2e) und einem die beiden Blattfedern an ihren hinteren Ende starr miteinander verbindenden Verbindungsteil (Schenkelverbindung 2d). In dieser Ausbildung kann der Fußeinsatz 2 aus Kunststoff hergestellt werden. Durch das verhältnismäßig weit nach vorn gezogene untere Ende 4 des starren Winkels 2a, 2b ergibt sich ein weitgehend natürlicher Bewegungsablauf für den Prothesenträger. Aufgrund der Formgebung des vorstehend beschriebenen Luftspaltes 7 wird bei zunehmendem Vorfußwinkel die Biegung des unteren Schenkels 2e nach vorn verlagert. Diese Biegung setzt zuerst ein in einem mittleren Bereich und wandert dann zu dem Anschlagbereich zwischen dem unteren Ende 4 des starren Winkels 2a, 2b und der oberen Auswölbung 5 des unteren Schenkels 2e und verlagert sich bei größerem Vorfußwinkel noch weiter nach vorn in einen Bereich des unteren Schenkels 2e, der bestimmungsgemäß einen sich nach vorn verjüngenden Querschnitt bzw. eine nach vorn abnehmende Materialstärke aufweist.

Um unter Belastung ein zu großes Aufspreizen des oberen Schenkels 2a gegenüber dem als Blattfeder wirkenden mittleren Schenkel 2c zu verhindern, ist in dem Ausführungsbeispiel gemäß den Figuren 1 bis 3 ein Biegebegrenzer vorgesehen. Dieser besteht aus einem Bolzen 9, der mit seinem unteren Ende am hinteren Ende des mittleren Schenkels 2c des Fußeinsatzes 2 befestigt ist, durch eine Ausnehmung im oberen Schenkel 2a relativ verschieblich geführt ist und an seinem anderen, den oberen Schenkel 2a überragenden Ende einen Anschlag 10 für den oberen Schenkel 2a aufweist.

Gemäß Figur 1 bildet der Prothesenkörper 1 eine einteilige, alle Funktionselemente umschließende kosmetische Hülle, die äußerlich keinerlei Befestigungselemente aufweist, sich bis in den Knöchelbereich erstreckt und oben bündig mit dem oberen Schenkel 2a abschließt. Diese kosmetische Hülle ist so ausgebildet, daß sie im letzten Bereich der Standphase eine teilweise Stützfunktion für den Fußeinsatz 2 bildet. Dieser Fußeinsatz 2 läßt sich einem Schuhspanner ähnlich unter Zuhilfenahme eines Spezialwerkzeuges in die kosmetische Hülle einschieben, wo sich der Fußeinsatz dann selbst arretiert und so ein unbeabsichtigtes Herausfallen aus der kosmetischen Hülle verhindert.

Die den Prothesenkörper 1 bildende kosmetische Hülle ist mit Ausnahme ihrer oberen Anschlußfläche vollständig von einer hautbildenden äußeren Kunststoffschaumschicht 11 ummantelt, die einen sich bis in den Zehenbereicht erstreckenden Innenfuß 12 aus Kunststoffschaum umschließt. Die äußere Kunststoffschaumschicht 11 weist eine spezifische Masse von etwa 0,4 g/cm³ und Rückstellkräfte auf, die klein sind gegenüber den Rückstellkräften des Innenfußes 12. Der den Innenfuß 12 bildende Kunststoffschaum weist eine spezifische Masse von 0,6 bis 1,0 g/cm³ auf.

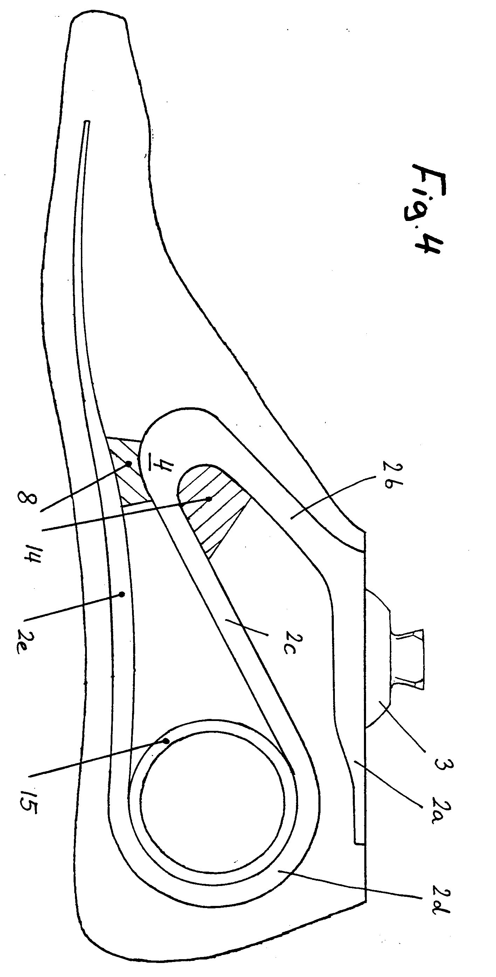

Figur 4 zeigt eine etwas abgewandelte Ausführungsform, bei der der Fußeinsatz 2 vorzugsweise aus einem Kohlefaserstoff besteht. In diesem Fall ist in dem zwischen dem vorderen Schrägschenkel 2b und dem mittleren Schenkel 2c eingeschlossenen keilförmigen Raum 13 des Fußeinsatzes 2 eine federelastische Abstützung 14 vorgesehen. Die hintere Schenkelverbindung 2d des Fußeinsatzes 2 umschließt eine rohrförmig ausgebildete Abstützung 15, die ggf. auch federelastische Eigenschaften aufweisen kann.

Bei beiden Ausführungsformen des Fußeinsatzes 2 ist der obere Schenkel 2a in einer Horizontalebene gegenüber dem unteren Schenkel 2e verwindbar, läßt also eine Torsion und dadurch eine Rotation des Prothesenfußes um eine vertikale Achse zu. Ferner ist der obere Schenkel 2a des Fußeinsatzes 2 in der lotrecht und quer zur Gehrichtung liegenden Frontalebene gegenüber dem unteren Schenkel 2e verwindbar und zwar zur Anpassung an Neigungen quer zur Gehrichtung.

## Patentansprüche

1. Gelenkloser Prothesenfuß mit einem innerhalb des Prothesenkörpers (1) vorgesehenen, die Prothesenbelastungen aufnehmenden und übertragenden federelastischen, einteilig ausgebildeten Fußeinsatz (2), der einen oberen horizontalen, den oberen Abschluß des Prothesenfußes bildenden und eine Verbindungsmöglichkeit mit der Prothese bietenden Schenkel (2a) sowie einen unteren Schenkel (2e) aufweist, der gegenüber dem oberen Schenkel (2a) verlängert ausgebildet ist und sich mit seinem freien Ende bis in den Fußspitzenbereich hinein erstreckt zur Erzielung einer hohen Vorfußelastizität in Verbindung mit einem hohen Energiespeichervermögen bei Vorfußbelastung, **dadurch gekennzeichnet,** daß der, zumindest eine Plantar- und Dorsalflexion sowie eine axiale Kompression ermöglichende Fußeinsatz (2) im Fußlängsschnitt eine angenähert S-förmige Ausbildung aufweist, wobei der genannte obere Schenkel (2a) mit einem sich unter einem stumpfen Winkel anschließenden vorderen Schrägschenkel (2b) ein insgesamt starres Winkelelement bildet, an dessen unteres Ende (4) sich ein mittlerer, blattfederähnlicher Schenkel (2c) anschließt, der an seinem hinteren Ende über eine angenähert halbkreisförmige Schenkelverbindung (2d) mit dem unteren Schenkel (2e) verbunden ist.

2. Prothesenfuß nach Anspruch 1, **dadurch gekennzeichnet,** daß sich das untere Ende (4) des starren Winkelelementes (2a, 2b) nach vorn bis in den Bereich der Zehengrundgelenke erstreckt.

3. Prothesenfuß nach Anspruch 1 oder 2, **dadurch gekennzeichnet**, daß der untere Schenkel (2e) des Fußeinsatzes (2) unterhalb des Anschlußbereiches des mittleren Schenkels (2c) am unteren Ende (4) des starren Winkels (2a, 2b) eine obere Auswölbung (5) aufweist, die mit der oberhalb von ihr liegenden Fläche (6) des genannten Anschlußbereiches einen sich nach vorn leicht keilfömig öffnenden Luftspalt (7) einschließt.

4. Prothesenfuß nach Anspruch 1, 2 oder 3, **dadurch gekennzeichnet,** daß der obere Schenkel (2a) des Fußeinsatzes (2) in einer Horizontalebene gegenüber dem unteren Schenkel (2e) verwindbar ist, also eine Torsion und dadurch eine Rotation des Prothesenfußes um eine vertikale Achse zuläßt.

5. Prothesenfuß nach einem der vorhergehenden Ansprüche, **dadurch gekennzeichnet**, daß der obere Schenkel (2a) des Fußeinsatzes (2) in der lotrecht und quer zur Gehrichtung liegenden Frontalebene gegenüber dem unteren Schenkel (2e) verwindbar ist zur Anpassung an Neigungen quer zur Gehrichtung.

6. Prothesenfuß nach einem der vorhergehenden Ansprüche, **dadurch gekennzeichnet**, daß die die Kraft-Weg- bzw. Winkel-Moment-Charakteristik der die genannten Verschwenkungen bzw. Verformungen bestimmenden federelastischen Eigenschaften (Federkennlinien) durch den S-fömigen Fußeinsatz (2) allein bestimmt sind.

7. Prothesenfuß nach einem der Ansprüche 1 bis 5, **dadurch** **gekennzeichnet**, daß die genannten Federkennlinien im Zusammenwirken des S-fömigen Fußeinsatzes (2) mit zusätzlichen federelastischen Komponenten (8, 14, 15) erzielt werden.

8. Prothesenfuß nach Anspruch 7, **dadurch gekennzeichnet**, daß sich das starre Winkelelement (2a, 2b) an seinem unteren Ende (4) über ein federelastisches Element (8) auf dem unteren Schenkel (2e) abstützt.

9. Prothesenfuß nach Anspruch 7 oder 8, **dadurch gekennzeichnet**, daß in dem zwischen dem vorderen Schrägschenkel (2b) und dem mittleren Schenkel (2c) eingeschlossenen keilförmigen Raum (13) des Fußeinsatzes (2) eine federelastische Abstützung (14) vorgesehen ist.

10. Prothesenfuß nach Anspruch 7, 8 oder 9, **dadurch gekennzeichnet,** daß die hintere Schenkelverbindung (2d) des Fußeinsatzes (2) eine ggf. federelastische Abstützung (15) umschließt.

11. Prothesenfuß nach Anspruch 10, **dadurch gekennzeichnet**, daß diese hintere Abstützung (15) rohrfömig ausgebildet ist.

12. Prothesenfuß nach einem der vorhergehenden Ansprüche, **dadurch gekennzeichnet**, daß für das freie Ende des oberen Schenkels (2a) des Fußeinsatzes (2) ein Biegebegrenzer (9, 10) vorgesehen ist.

13. Prothesenfuß nach Anspruch 12, **dadurch gekennzeichnet**, daß der Biegebegrenzer (9, 10) ein Bolzen (9) o. dgl. ist, der mit seinem unteren Ende am hinteren Ende des mittleren Schenkels (2c) des Fußeinsatzes (2) befestigt ist, durch eine Ausnehmung im oberen Schenkel (2a) relativ verschieblich geführt ist und an seinem anderen, den oberen Schenkel (2a) überragenden Ende einen Anschlag (10) für den oberen Schenkel (2a) aufweist.

14. Prothesenfuß nach einem der vorhergehenden Ansprüche, **dadurch gekennzeichnet**, daß der obere Schenkel (2a) des Fußeinsatzes (2) einen Fußadapter (3) trägt.

15. Prothesenfuß nach einem der vorhergehenden Ansprüche, **dadurch gekennzeichnet**, daß die untere Kontur des unteren horizontalen Schenkels (2e) des Fußeinsatzes (2) an die Kontur der Fußunterseite angepaßt ist.

16. Prothesenfuß nach einem der vorhergehenden Ansprüche, **dadurch gekennzeichnet**, daß der Prothesenkörper (1) aus einer alle Funktionselemente einbettenden fußfömigen Umschäumung besteht.

17. Prothesenfuß nach einem der vorhergehenden Ansprüche, **dadurch gekennzeichnet**, daß der Prothesenkörper (1) eine einteilige, alle Funktionselemente umschließende kosmetische Hülle bildet.

18. Prothesenfuß nach Anspruch 17, **dadurch gekennzeichnet**, daß der Prothesenkörper (1) mit Ausnahme seiner oberen Anschlußfläche vollständig von einer hautbildenden äußeren Kunststoffschaumschicht (11) ummantelt ist, die einen sich bis in den Zehenbereich erstreckenden Innenfuß (12) aus Kunststoffschaum umschließt.

19. Prothesenfuß nach Anspruch 18, **dadurch gekennzeichnet**, daß die äußere Kunststoffschaumschicht (11) eine spezifische Masse von etwa 0,4 g/cm³ und Rückstellkräfte aufweist, die kleiner sind gegenüber den Rückstellkräften des Innenfußes (12).

20. Prothesenfuß nach Anspruch 18 oder 19, **dadurch gekennzeichnet,** daß der den Innenfuß (12) bildende Kunststoffschaum eine spezifische Masse von 0,6 bis 1,0 g/cm³ aufweist.

## Claims

1. Non-jointed prosthetic foot with a one-piece resilient foot insert (2) which is provided within the body (1) of the prosthesis and receives and transfers the prosthesis loads, and which has an upper, horizontal section (2a) which forms the upper termination of the prosthetic foot and provides a possibility for connection to the prosthesis, as well as a lower section (2e) which is elongated with respect to the upper section (2a) and with its free end extends into the region of the point of the foot in order to achieve high elasticity in the front region of the foot in conjunction with a high energy storage capacity when there is a load on the front region of the foot, characterized in that the foot insert (2), which allows at least a plantar and dorsal flexion as well as an axial compression, has an approximately S-shaped construction in the longitudinal section of the foot, the said upper section (2a) forming with a front oblique section (2b) adjoining it at an obtuse angle a generally rigid angular element, whereof the lower end (4) is adjoined by a central, leafspring-like section (2c) which is connected at its rear end by way of an approximately semi-circular section connection (2d) to the lower section (2e).

2. Prosthetic foot according to Claim 1, characterized in that the lower end (4) of the rigid angular element (2a, 2b) extends forwards as far as the region of the toe base joints.

3. Prosthetic foot according to Claim 1 or 2, characterized in that the lower section (2e) of the foot insert (2) has below the connection region of the central section (2c) to the lower end (4) of the rigid angular piece (2a, 2b) an upper convex elevation (5) which, along with the surface (6), located above it, of the said connection region, encloses an air gap (7) which opens forwards in a slightly wedge-shaped manner.

4. Prosthetic foot according to Claim 1, 2 or 3, characterized in that the upper section (2a) of the foot insert (2) is twistable with respect to the lower section (2e) in a horizontal plane, and thus allows torsion and consequently rotation of the prosthetic foot about a vertical axis.

5. Prosthetic foot according to one of the preceding claims, characterized in that the upper section (2a) of the foot insert (2) is twistable with respect to the lower section (2e) in the frontal plane located perpendicularly and transversely with respect to the walking direction in order to adapt to inclinations transverse with respect to the walking direction.

6. Prosthetic foot according to one of the preceding claims, characterized in that the resilient properties (spring characteristics) determining the force/displacement or angle/moment characteristic of the said tilting or deformations are determined solely by the S-shaped foot insert (2).

7. Prosthetic foot according to one of Claims 1 to 5, characterized in that the said spring characteristics are achieved by the cooperation of the S-shaped foot insert (2) with additional resilient components (8, 14, 15).

8. Prosthetic foot according to Claim 7, characterized in that the rigid angular element (2a, 2b) is supported at its lower end (4) on the lower section (2e) by way of a resilient element (8).

9. Prosthetic foot according to Claim 7 or 8, characterized in that there is provided in the wedge-shaped space (13) enclosed between the front oblique section (2b) and the central section (2c) a resilient support (14).

10. Prosthetic foot according to Claim 7, 8 or 9, characterized in that the rear section connection (2d) of the foot insert (2) encloses an optionally resilient support (15).

11. Prosthetic foot according to Claim 10, characterized in that said rear support (15) is of tubular construction.

12. Prosthetic foot according to one of the preceding claims, characterized in that a flexion limiter (9, 10) is provided for the free end of the upper section (2a) of the foot insert (2).

13. Prosthetic foot according to Claim 12, characterized in that the flexion limiter (9, 10) is a pin (9) or the like which is secured by means of its lower end to the rear end of the central section (2c) of the foot insert (2), is guided relatively displaceably through a cutout in the upper section (2a) and has at its other end, which projects beyond the upper section (2a), a stop (10) for the upper section (2a).

14. Prosthetic foot according to one of the preceding claims, characterized in that the upper section (2a) of the foot insert (2) bears a foot adapter (3).

15. Prosthetic foot according to one of the preceding claims, characterized in that the lower contour of the lower horizontal section (2e) of the foot insert (2) is matched to the contour of the underside of the foot.

16. Prosthetic foot according to one of the preceding claims, characterized in that the body (1) of the prosthesis comprises a foot-shaped foam material in which all the functional elements are embedded.

17. Prosthetic foot according to one of the preceding claims, characterized in that the body (1) of the prosthesis forms a one-piece cosmetic sheath enclosing all the functional elements.

18. Prosthetic foot according to Claim 17, characterized in that the body (1) of the prosthesis, with the exception of its upper connection surface, is completely encased by an outer plastics foam layer (11) which forms a skin and which encloses an inner foot (12) of plastics foam extending as far as the toe region.

19. Prosthetic foot according to Claim 18, characterized in that the outer plastics foam layer (11) has a specific mass of approximately 0.4 g/cm³ and restoring forces which are smaller than the restoring forces of the inner foot (12).

20. Prosthetic foot according to Claim 18 or 19, characterized in that the plastics foam forming the inner foot (12) has a specific mass of 0.6 to 1.0 g/cm³.

## Revendications

1. Prothèse de pied, non articulée, avec, dans le corps de prothèse (1), un insert de pied (2), réalisé d'un seul tenant, supportant et transmettant les sollicitations exercées sur la prothèse et présentant l'élasticité d'un ressort, comportant une branche supérieure (2a) horizontale, constituant la délimitation supérieure de la prothèse de pied et offrant une possibilité de liaison à la prothèse, ainsi qu'une branche inférieure (2e), réalisée prolongée par rapport à la branche supérieure (2a), et s'étendant, par son extrémité libre, jusque dans la zone de la pointe de pied, en vue d'obtenir une bonne élasticité pour l'avant-pied, en liaison avec une capacité de stockage d'énergie élevée en cas de sollicitation de l'avant-pied, caractérisée en ce que l'insert de pied (2), permettant au moins une flexion plantaire et une flexion dorsale, ainsi qu'une compression axiale, présente, en coupe longitudinale du pied, une configuration à peu près en S, ladite branche supérieure (2a) constituant, à l'aide d'une branche oblique avant (2b), se raccordant à ladite branche supérieure (2a) en faisant un angle obtus, un élément coudé, globalement rigide, à l'extrémité inférieure (4) duquel se raccorde une branche médiane (2c) semblable à un ressort à lame, reliée par son extrémité arrière à la branche inférieure (2e), par l'intermédiaire d'une liaison de branches (2d) à peu près en forme de demi-cercle.

2. Prothèse de pied selon la revendication 1, caractérisée en ce que l'extrémité inférieure (4) de l'élément coudé (2a, 2b) s'étend vers l'avant, jusqu'à la zone de l'articulation du creux entre orteils.

3. Prothèse de pied selon la revendication 1 ou 2, caractérisée en ce que la branche inférieure (2e) de l'insert de pied (2) présente, au-dessous de la zone de raccordement de la branche médiane (2c), à l'extrémité inférieure (4) de l'élément coudé (2a, 2b) rigide, une courbure supérieure (5), faisant avec la surface (6), située au-dessus d'elle, de ladite zone de raccordement un entrefer (7), allant en s'ouvrant légèrement en forme de coin, en direction de l'avant.

4. Prothèse de pied selon la revendication 1, 2 ou 3, caractérisée en ce que la branche supérieure (2a) de l'insert de pied (2) est déformable, dans un plan horizontal, par rapport à la branche inférieure (2e), en permettant donc une torsion et, de ce fait, une rotation de la prothèse de pied autour d'un axe vertical.

5. Prothèse de pied selon l'une des revendications précédentes, caractérisée en ce que la branche supérieure (2a) de l'insert de pied (2) est déformable, dans le plan frontal, passant par la verticale et transversalement par rapport à la direction de la marche, en vue d'assurer une adaptation à des inclinaisons subies transversalement par rapport à la direction de la marche.

6. Prothèse de pied selon l'une des revendications précédentes, caractérisée en ce que la caractéristique force-déplacement, respectivement angle-couple des propriétés d'élasticité d'un ressort (caractéristiques d'élasticité) déterminant lesdits pivotements et/ou déformations sont déterminées uniquement par l'insert de pied (2) en S.

7. Prothèse de pied selon l'une des revendications 1 à 5, caractérisée en ce que lesdites caractéristiques d'élasticité sont obtenues par coopération de l'insert de pied (2) en S avec des composants (8, 14, 15) supplémentaires, ayant l'élasticité de ressorts.

8. Prothèse de pied selon la revendication 7, caractérisée en ce que l'élément coudé (2a, 2b) prend appui à son extrémité inférieure (4) sur la branche inférieure (2e), par l'intermédiaire d'un élément (8) ayant l'élasticité d'un ressort.

9. Prothèse de pied selon la revendication 7 ou 8, caractérisée en ce qu'un appui (14) ayant l'élasticité d'un ressort est prévu dans l'espace cunéiforme (13), enclos entre la branche avant (2b) oblique et la branche médiane (2c), de l'insert de pied (2).

10. Prothèse de pied selon la revendication 7, 8 ou 9, caractérisée en ce que la liaison de branches (2d) arrière de l'insert de pied (2) entoure un appui (15) ayant, le cas échéant, l'élasticité d'un ressort.

11. Prothèse de pied selon la revendication 10, caractérisée en ce que cet appui arrière (15) est tubulaire.

12. Prothèse de pied selon l'une des revendications précédentes, caractérisée en ce qu'un limiteur de flexion (9, 10) est prévu pour l'extrémité libre de la branche supérieure (2a) de l'insert de pied (2).

13. Prothèse de pied selon la revendication 12, caractérisée en ce que le limiteur de flexion (9, 10) est un boulon (9) ou analogue, fixé par son extrémité inférieure sur l'extrémité arrière de la branche médiane (2c) de l'insert de pied (2), guidé, pour permettre un déplacement radial relatif, au moyen d'un évidement ménagé dans la branche supérieure (2a), et présentant à son autre extrémité, dépassant de la branche supérieure (2a), une butée (10) pour la branche supérieure (2a).

14. Prothèse de pied selon l'une des revendications précédentes, caractérisée en ce que la branche supérieure (2a) de l'insert de pied (2) porte un adaptateur de pied (3).

15. Prothèse de pied selon l'une des revendications précédentes, caractérisée en ce que le contour inférieur de la branche inférieure (2e) horizontale de l'insert de pied (2) est adapté au contour de la face inférieure du pied.

16. Prothèse de pied selon l'une des revendications précédentes, caractérisée en ce que le corps de prothèse (1) est composé d'un enrobage en matériau alvéolaire, ayant la forme d'un pied, dans lequel sont noyés tous les éléments fonctionnels.

17. Prothèse de pied selon l'une des revendications précédentes, caractérisée en ce que le corps de prothèse (1) constitue une enveloppe cosmétique, d'une seule pièce, enclosant tous les éléments fonctionnels.

18. Prothèse de pied selon la revendication 17, caractérisée en ce que le corps de prothèse (1) est complètement enveloppé, à l'exception de sa surface de raccordement supérieure, d'une couche extérieure (11) en matériau synthétique alvéolaire, constituant une peau, enclosant un pied intérieur (12) en matière synthétique alvéolaire, s'étendant jusque dans la zone des orteils.

19. Prothèse de pied selon la revendication 18, caractérisée en ce que la couche en matière synthétique alvéolaire (11) a une masse spécifique d'à peu près 0,4 g/cm³ et présente des forces de rappel inférieures aux forces de rappel du pied intérieur (12).

20. Prothèse de pied selon la revendication 18 ou 19, caractérisée en ce que la matière synthétique alvéolaire constituant le pied intérieur (12) a une masse spécifique comprise dans la plage allant de 0,6 à 1,0 g/cm³.
